# EUROPEAN PATENT APPLICATION

(11) **EP 2 462 968 A1**
(43) Date of publication of application: **13.06.2012**
(21) Application number: 06794027.0
(22) Date of filing: 15.06.2006
(51) Int. Cl.: A61M 5/315, A61M 5/31

(54) **INJECTOR DEVICE FOR SYRINGES**

(30) Priority: 05.07.2005 ES 200501550 U
(71) Applicant: Innovaciones en Suministro de Nutrición Enteral, S.L., 46910 Benetusser (Valencia) (ES)
(72) Inventor: PRIETO JIMÉNEZ, Luis, E-46910 Alfafar (Valencia) (ES)
(74) Representative: Gallego Jiménez, José Fernando
(86) International application number: PCT/ES2006/000352
(87) International publication number: WO 2007/003669

(57) **Abstract**

The invention relates to an injector device for syringes, which enables the syringe plunger to be moved gradually with little effort on the part of the patient or a third person. The invention comprises: a first support (5) which is equipped with a cavity (6) in which the barrel of the syringe (1) is inserted perpendicularly to the first support (5), the flanges (2) of the syringe resting on the peripheral edge of the cavity (6); and a second central support (9) on which the syringe plunger (3) rest. The aforementioned second support (9) is guided manually along parallel guides (8) that extend from the first support (5), between said first support (5) and the ends of the parallel guides (8), such as to push the plunger (3) inside the cylindrical barrel (1).

## Description

### OBJECT OF THE INVENTION

The present invention relates to an application for syringes, particularly for those syringes used to introduce fluids of a certain density into the body.

The object of the invention is to shape the device with a simple structure, manually operable, and that is provided with means to facilitate the gradual movement of the syringe plunger with barely any effort required on the part of the patient or a third person.

### PRIOR ART OF THE INVENTION

Due to an ageing population and developments in the field of medicine, the situation has arisen in which the need for sanitary assistance at home is increasingly greater.

One of the complications or consequences of a wide group of illnesses consists in the alteration of deglutition or dysphagia, which determines the need to place, for example, nasogastric tubes that guarantee that the patients are fed. Illnesses that can cause dysphagia include, amongst others, degenerative illnesses such as dementia, patients having undergone a tracheotomy, neuromuscular and oncological illnesses,...

In the tube feeding process, the ground up food is generally administered through the tube with a density higher than that of water, syringes of considerable dimensions thus being used for this purpose. For this reason, the pressure required to move the syringe plunger in order to be able to inject the food through the nasogastric tube is very high. This can consequently generate great difficulty for the patient due to the force that must be exerted on the transversal flanges of the syringe that is already of a reduced size. Furthermore, if we add the fact that this concerns procedures that are carried out on a daily basis and several times per day, repeated microtraumas may be caused that will have clinical repercussions over time and moreover that will have a far greater probability of occurring in the case of individuals with rheumatic processes. Furthermore, the pain it can cause to a patient's fingers and hand must be taken into consideration.

Likewise, there are cases of advanced ailments that require the participation of an assistant to provide the patient with the diet required.

Certain patents related to the technical domain of this invention are described in the following.

The Invention Patent ES 2 176 457 describes a syringe for the dosed injection of a medicine which intends to facilitate the operation of said syringe through the use of an interchangeable cartridge, of the type that contains a piston forced into a tubular cartridge in order to eject a dose corresponding to the movement of the piston.

The Invention Patent ES 2 128 694 describes an injector, operated mechanically and controlled manually, that incorporates a pistol-type handle and houses in its interior a motor associated to a screw that determines the movement of the syringe plunger.

The Invention Patent ES 1 055 061 refers to a syringe that includes a cylinder that houses a plunger that is axially displaceable in said cylinder, related by means of one or more elastic traction elements that constantly drive said plunger towards the cylinder base. The distance between the anchorage points of the piston where the elastic elements are anchored is variable, offering the possibility of selecting different anchorage points that produce a different extension of the elastic elements and therefore different pushing pressures.

### DESCRIPTION OF THE INVENTION

The present invention relates to an injector device for syringes that aims to reduce the force exerted by the fingers when moving the plunger of a syringe by incorporating a mechanism for such a purpose that offers a greater support surface so that in this way it is possible to reduce the application pressure necessary, without straining the hand or the joints, and to allow the patient him/herself to inject him/herself in a way simple without the need for external help.

The injector device consists of a first support and a second support that moves in a guided way on the parallel guides with respect to the first support.

The first support presents a cavity that is crossed by the cylindrical barrel of the syringe, said cavity being delimited by a peripheral edge on which the flanges that make up the base of the barrel of the syringe rest. This cavity can consist, in a possible embodiment, in a U-shaped section made on one side of the first support or, for example, it can be a circular opening through which the barrel of the syringe is introduced by its tip and that presents a slightly larger diameter than that of the barrel.

The base of the plunger or push flange rests on one of the faces of the second moveable support, in this way being arranged so that when pressure is applied with the hand on said second support, while holding the fixed position of the first support, the plunger is forced to advance and will gradually penetrate the cylinder pushing the viscous fluid contained in its interior in the direction towards the tube, for example. The wide surface with which the second support is provided allows both hands to be applied on said second support with the thumbs supported on the external face of the first support, directing the plunger with barely any resistance towards the interior of the cylinder, thus proving particularly useful for fluids of a certain density.

The maximum run of the second support is defined by the distance existing between the first support and the final end of the parallel guides that start at said first support. Optionally, provision has been made for the second support to incorporate, into its wall opposite the first support, a rebate into which the base of the plunger or push flange is inserted, in this way facilitating positional stability during the run of the plunger and avoiding undesired movement.

Respective stoppers are found on the final ends of the parallel guides that will prevent the escape of the second support and will determine its maximum run as mentioned previously, said stoppers could be substituted with a third support.

Therefore, in accordance with this structuring of the device proposed, it is possible for the patient to introduce dense food by means of this syringe towards a tube while only exerting minimum pressure.

### DESCRIPTION OF THE DRAWINGS

In order to complement the description being given and with the object of aiding the better comprehension of the characteristics of the invention, in accordance with a preferred example of a practical embodiment of the latter, a set of drawings is included as an integral part of said description which is of an illustrative and not limitative character, in which the following is disclosed:
Figure 1. - Shows a perspective view in which it is possible to see the device that is the object of the invention without the syringe.
Figure 2. - Shows a perspective view of the injector device in which the syringe is seen mounted for the maximum extension position of the plunger.
Figure 3. - Shows a perspective view of the injector device for an intermediate position during the compression of the syringe plunger by means of the action of the injector device.
Figure 4.- Shows a perspective view of a feature of the injector device with the syringe mounted, in which is it possible to see the flanges of the syringe resting on the peripheral edge of the section of the first support.

### PREFERRED EMBODIMENT OF THE INVENTION

With reference to the Figures a description is given hereafter of a preferred embodiment of the injector device for syringes that constitutes the object of the invention, in an application for syringes that conventionally consist of an essentially cylindrical barrel (1), provided with at least one flange (2) on its base, on the interior of which a plunger (3) is moved that has a push flange (4) at its base.

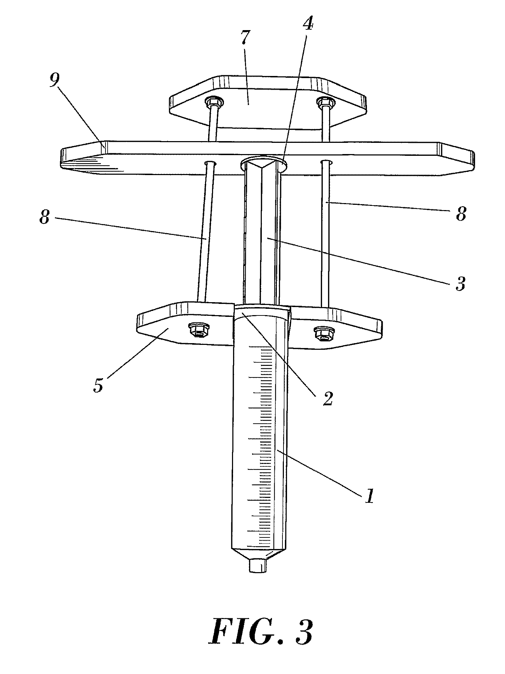

As can be seen in Figure 3 the injector device is made up of:
- a first support (5) provided with a cavity (6) in which the barrel of the syringe (1) is inserted perpendicularly to said first support (5), said syringe resting by means of the flanges (2) on the peripheral edge of said cavity (6) on a internal wall of the first support (5),
- a second support (9) against which the push flange (4) of the syringe rests and that moves guided by manual action on some parallel guides (8), that start from the first support (5), between said first support (5) and the final ends of said parallel guides (8) thus pushing the plunger (3) in the interior of the cylindrical barrel (1).

The final ends of the parallel guides (8) can be finished with respective stoppers that prevent the accidental escape of the second support (9) by delimiting its maximum run or alternatively a third support (7) parallel to the first support (5) can be coupled to said final ends.

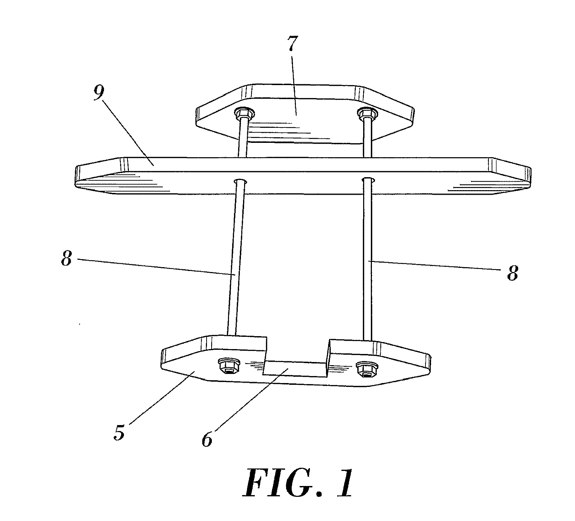

The cavity (6) can consist, as represented in Figure 1, of a U-shaped section made on one of the sides of the first support (5).

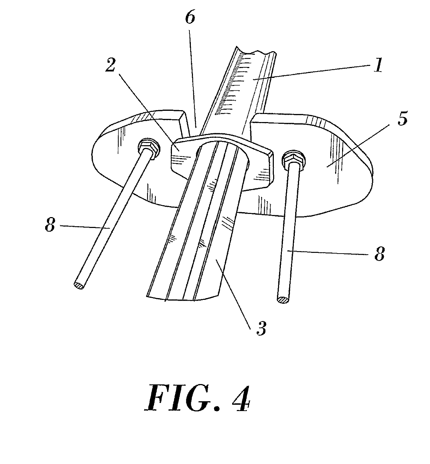

In Figure 4 it is possible to observe the way in which the barrel (1) of the syringe supported by its flanges (2) on the first support (5) is positioned.

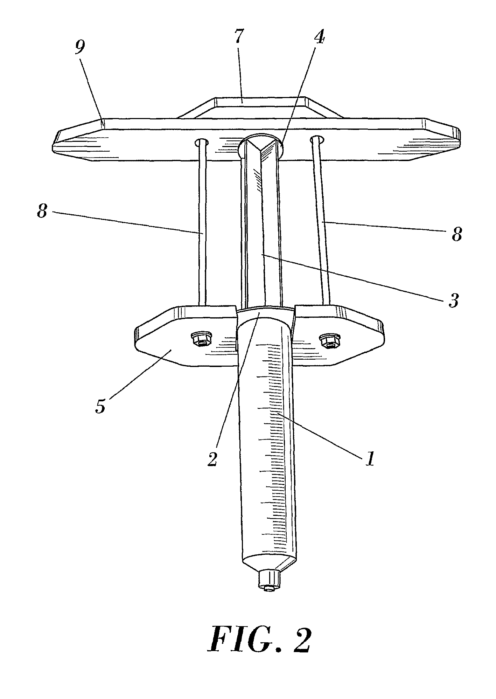

In Figures 2 and 3 it is possible to see two working sequences of the device that is the object of this invention, in Figure 2 the plunger (3) appears completely extended, and then, as pressure is exerted on the second support (9) by means of the use of the hands, holding the first support in a fixed position (5), said second support (9) is moved with very little effort to push the plunger (3) towards the interior of the barrel (1), as can be seen in Figure 3.

## Claims

1. Injector device for syringes, in an application for syringes that consist of an essentially cylindrical barrel (1), provided with at least one lateral flange (2) on its base and with a tip on its end, in the interior of which a plunger is moved (3) that has a push flange (4) on its base, **characterised in that** includes:
- a first support (5) provided with a cavity (6) in which the barrel of the syringe (1) is inserted perpendicularly to said first support (5), said syringe resting by means of the flanges (2) on the peripheral edge of said cavity (6) on a internal wall of the first support (5),
- a second support (9) against which the push flange (4) of the syringe rests and that moves guided by manual action on some parallel guides (8), that start from the first support (5), between said first support (5) and the final ends of said parallel guides (8) thus pushing the plunger (3) in the interior of the cylindrical barrel (1).

2. Injector device for syringes according to claim 1 **characterised in that** the cavity (6) has a U-shaped section made on one of the sides of the first support (5).

3. Injector device for syringes according to claim 1 **characterised in that** the cavity (6) consists of a circular opening with a diameter slightly greater than the diameter of the barrel (1) of the syringe that is introduced through the latter by its tip.

4. Injector device for syringes according to claim 1 **characterised in that** the second support (9) incorporates into its wall, opposite the first support (5), a rebate into which the push flange (4) is inserted thus facilitating the positional stability of the plunger (3) during its run.

5. Injector device for syringes according to claim 1 **characterised in that** the final ends of the parallel guides (8) are finished with respective stoppers.

6. Injector device for syringes according to claim 1 **characterised in that** a third support (7) parallel to the first support (5) is coupled to the final ends of the parallel guides (8).
